# EUROPÄISCHE PATENTSCHRIFT

(11) **EP 2 390 664 B1**
(45) Veröffentlichungstag und Bekanntmachung des Hinweises auf die Patenterteilung: **17.04.2013**
(21) Anmeldenummer: 10005425.3
(22) Anmeldetag: 25.05.2010
(51) Int. Cl.: G01N 33/542, G01N 33/543, G01N 33/74

(54) **Verfahren zur elektrochemischen Detektion von Bindungsreaktionen**
Method for electrochemical detection of binding reactions
Procédé de détection électrochimique de réactions de liaison

(43) Veröffentlichungstag der Anmeldung: 30.11.2011
(73) Patentinhaber: Fraunhofer-Gesellschaft zur Förderung der angewandten Forschung e.V., 80686 München (DE)
(72) Erfinder: Ettlinger, Julia, 14550 Gross Kreutz (DE); Gajovic-Eichelmann, Nenad, 14089 Berlin (DE); Micheel, Burkhard, 17268 Gerswalde (DE); Scharte, Gudrun, 13189 Berlin (DE); Schenk, Jörg, 10439 Berlin (DE)
(74) Vertreter: Katzameyer, Michael

(56) Entgegenhaltungen:
- US-A1- 2004 020 791
- GHINDILIS A L ET AL: "Nanomolar determination of the ferrocene derivatives using a recycling enzyme electrode: Development of a redox label immunoassay" ANALYTICAL LETTERS, Bd. 28, Nr. 1, 1995, Seiten 1-11, XP008124810 ISSN: 0003-2719
- SAPIN RÉMY ET AL: "Efficacy of a new blocker against anti-ruthenium antibody interference in the Elecsys free triiodothyronine assay." CLINICAL CHEMISTRY AND LABORATORY MEDICINE : CCLM / FESCC 2007 LNKD- PUBMED:17378744, Bd. 45, Nr. 3, 2007, Seiten 416-418, XP008124809 ISSN: 1434-6621
- FORROW NIGEL J ET AL: "Synthesis, characterization, and evaluation of ferrocene-theophylline conjugates for use in electrochemical enzyme immunoassay." BIOCONJUGATE CHEMISTRY, Bd. 15, Nr. 1, Januar 2004 (2004-01), Seiten 137-144, XP002593998 ISSN: 1043-1802
- WEI M-Y ET AL: "Development of redox-labeled electrochemical immunoassay for polycyclic aromatic hydrocarbons with controlled surface modification and catalytic voltammetric detection" BIOSENSORS AND BIOELECTRONICS 20090515 ELSEVIER LTD; ELSEVIER ADVANCED TECHNOLOGY; THE BOULEVARD GB, Bd. 24, Nr. 9, 15. Mai 2009 (2009-05-15), Seiten 2909-2914, XP002593999 DOI: DOI:10.1016/J.BIOS.2009.02.031

## Beschreibung

Die vorliegende Erfindung betrifft ein Verfahren zur direkten, hoch empfindlichen elektrochemischen Detektion von Antikörper-Antigen Bindungsreaktionen und anderen biochemischen Interaktionen in Lösung. Das Verfahren eignet sich für wasch- und separierungsfreie Immunoassays in der Diagnostik, insbesondere für automatisierte und mikrofluidische Immunoassays. Das Verfahren eignet sich auch für preiswerte Immunoassaysysteme und Teststreifen für den Einmalgebrauch.

Immunoassays nutzen die Antigen-Antikörper-Reaktion zur spezifischen Detektion geringster Konzentrationen eines Analyten in komplexen Medien wie Blut, Serum, Urin oder Lebensmitteln. In der klinischen Diagnostik, Umwelt- und Lebensmittelanalytik sind Immunoassays seit Jahrzehnten unverzichtbare Werkzeuge für die Detektion niedrigster Konzentrationen von Hormonen, Metaboliten, Proteinen, Biomarkern, Toxinen, Pestiziden, pathogenen Bakterien und Viren. Viele der wichtigsten klinisch-diagnostischen Analyten sind nur mit Immunoassays preiswert und schnell messbar, da es keine alternativen analytisch-chemischen Verfahren gibt, die eine gleichwertige Kombination aus hoher Sensitivität (Nachweis von nanomolaren oder niedrigeren Konzentrationen) und hoher Spezifität (Nachweis des Analyten in Gegenwart von chemisch ähnlich aufgebauten Störsubstanzen) bieten. Alternative Verfahren wie die Chromatografie (z.B. HPLC, Gaschromatografie) bedürfen einer oft mehrstufigen Probenvorbereitung, z.B. durch Extraktion und chemische Derivatisierung.

Seit der Erfindung des Immunoassays vor 50 Jahren durch Rosalyn Yalow und Salomon Berson wurde eine Vielzahl an unterschiedlichen Ausführungsformen entwickelt, die als verschiedene Immunoassayformate bezeichnet werden. Die grundlegende Unterscheidung von heterogenen Formaten, wie dem ELISA (enzyme linked immunosorbent assay) und homogenen Formaten erfolgt anhand des Aggregatszustandes der zur Detektion genutzten Antikörper: Bei den heterogenen Assays wird der Antikörper (seltener das Antigen) an einer Oberfläche immobilisiert (Reaktionsröhrchen, Mikrotiterplatte, Teststreifen), sämtliche Reaktionsschritte wie Wasch-und Separationsschritte sowie die Detektion erfolgen ebenfalls an der Oberfläche. Bei den homogenen Formaten wird keiner der Bindungspartner immobilisiert, es sind keine Wasch- und Separationsschritte nötig, die Detektion erfolgt ebenfalls in Lösung.

Eine andere Unterscheidungsmöglichkeit ergibt sich anhand der verwendeten Detektionsmethode. Da die Antikörper-Antigen-Bindungsreaktion nicht direkt sichtbar gemacht werden kann, muss einer der Bindungpartner chemisch mit einem molekularen Marker oder "Label" gekoppelt werden (Ausnahme: Massensensitive Biosensoren auf der Basis von Oberflächenplasmonresonanz, Gitterkoppler, Quarz-Crystal Microbalance, Surface acoustic waves und ähnlichen Techniken messen direkt die molekulare Bindungreaktion in Echtzeit, wenn einer der Bindungpartner auf der sensitiven Oberfläche immobilisiert ist. Diese Verfahren werden jedoch bisher nur in Forschungsanwendungen, nicht in der Routineanalytik eingesetzt). Ein geeignetes Label muss eine hohe "spezifische Aktivität" haben, d.h. es muss pro Labelmolekül möglichst viele Signalereignisse produzieren. Zu den am häufigsten genutzten Labels gehören radioaktive Isotope (Radioimmunoassay, "RIA"), Fluoreszenzfarbstoffe (Fluorescein, Rhodamine usw.), fluoreszente Halbleiternanopartikel ("Quantum dots"), Polymernanopartikel ("Latex beads", Agglomerationsimmmunoassay) und Enzyme wie Peroxidase, zusammen mit einem kolorimetrischen, fluorogenen oder chemolumineszenten Enzymsubstrat (ELISA). Die höchste spezifische Aktivität, die sogar den Nachweis eines einzelnen Labelmoleküls ermöglicht, besitzen radioaktive Isotope. Aufgrund der Gesundheitsgefährdung, die von Radioaktivität ausgeht, der damit verbundenen hohen Anforderungen an das Labor ("Isotopenlabors") und den hohen Kosten für die Entsorgung der Rückstände, werden Radioaktivimmunoassays zunehmend von alternativen Verfahren wie dem ELISA ersetzt.

Eine weitere Unterscheidung zwischen verschiedenen Immunoassays ergibt sich anhand der Art der verwendeten Antikörper. Antikörper sind komplex aufgebaute Proteine mit konstanten Bereichen, die die Struktur determinieren und bei allen Antikörpernklassen ähnlich zusammengesetzt sind, und hoch variablen Bereichen, die die Antigen-Bindungsstellen bilden. Die ursprünglich benutzten polyklonalen Seren, die eine Vielzahl von Antikörpern mit variabler Spezifität und Affinität enthalten, werden in vielen Anwendungen von monoklonalen Antikörpern ersetzt, die nur genau eine Antikörper-Entität ("Klon") enthalten, mit wohl definierter Spezifität und Affinität. Monoklonale Antikörper können theoretisch in beliebigen Mengen mit identischen Eigenschaften hergestellt werden. Daneben wurden für manche Analysen auch sog. Antikörper-Fragmente verwendet, die durch enzymatischen Verdau aus ganzen Antikörpern hergestellt werden können. Single-Chain-Antikörper sind rekombinante Antikörper-Fragmente, die durch Methoden des Genetic Engineering hergestellt werden können. Prinzipiell können alle Arten von Antikörpern und daraus abgeleiteten molekularen Bindern in den bekannten Immunoassayformaten genutzt werden.

Zu den bekanntesten klinischen Analyten, die mit Immunoassays nachgewiesen werden, gehören hCG (Schwangerschaftstest), Thyroid-Hormone wie TSH (Schilddrüsenerkrankungen), Steroidhormone (Endokrinologie, Fruchtbarkeit) und PSA (Biomarker für Prostatakarzinom).

Man kann zusammenfassend sagen, dass Immunoassays auf der Basis von monoklonalen Antikörpern oder polyklonalen Antiseren zu den wichtigsten analytisch-chemischen Verfahren der Biotechnologie, klinischen Diagnostik, Umwelt- und Lebensmittelanalytik zählen und einen hohen kommerziellen Wert besitzen.

Im Stand der Technik sind eine Vielzahl von Methoden beschrieben, um einen Immunoassay durchzuführen. Die meisten heterogenen Immunoassays erfordern mehrere manuelle Arbeitsschritte wie z.B. Pipettiervorgänge, Probenverdünnung, Waschvorgänge, die einem genauen zeitlichen Protokoll folgen müssen. Daher ist in der Regel geschultes Personal und ein dafür speziell ausgerüstetes Labor nötig, um solche Assays korrekt durchzuführen. Die erforderlichen Detektionsgeräte ("ELISA-Reader", Mikrotiterplattenreader, Immunoassayanalysator) sind teuer und nicht portabel. So werden z.B. in den Patentschriften [US4016043A, US3839153A] Protokolle zur Durchführung eines ELISA-Tests beschrieben. Der Fachmann erkennt leicht, dass derart komplexe Analysenabläufe nur von geschultem Personal und nur in einer geeigneten Laborumgebung durchgeführt werden können. Die Automatisierung solcher Abläufe ist sehr aufwendig und erfordert hoch komplexe Automaten.

Für den Vor-Ort-Einsatz hat sich das heterogene Lateral-Flow Immunoassayformat durchgesetzt ("Teststreifen"-Immunoassay). US6156271A beschreibt eine moderne Variante dieses Assayformats. Dieser Test läuft nach Zugabe der Probenlösung von alleine ab, und die Detektion erfolgt rein visuell (Ablesen des Vorhandensein von einer oder zwei farbigen Banden auf dem Teststreifen durch den Nutzer). Es ist offensichtlich, dass ein solches Ausleseverfahren keine quantitativen Ergebnisse (d.h. genaue Konzentrationsmessungen) ergeben kann, sondern dass nur eine Ja/Nein-Aussage oder eine semi-quantitative Aussage (Konzentration liegt unter/über einem bestimmten Schwellenwert) möglich sind.

Dem Fachmann ist bekannt, dass homogene Immunoassays dagegen besonders geeignet sind, um schnelle, quantitative und voll automatisierte Immunoassays zu realisieren. Bei homogenen Immunoassays erfolgt die Signalgenerierung zeitgleich mit der Bindungsreaktion. Im Gegensatz zu den oben beschriebenen heterogenen Immunoassays bestehen homogene Immunoassays zumeist nur aus einer oder wenigen Dosieroperationen, einer Inkubationszeit und dem Detektionsschritt. Im idealen Fall ist nur die Mischung der Probe und eines fertigen Reagenziengemisches nötig, bevor ein Endwert gemessen werden kann (sog. "Mix-and-Measure" Tests). Bei einer 1:1 Mischung, d.h. gleichen Volumenanteilen Probe und Reagenziengemisch ist die Dosierung mit einfachsten Mitteln mit hoher Präzision möglich.

Es ist dem Fachmann leicht ersichtlich, dass ein homogener Immunoassay auch die kürzest-mögliche Analysendauer erlaubt, da die Messung sofort nach dem Erreichen des Bindungsgleichgewichts zwischen Antikörper und Antigen möglich ist.

Ein Nachteil von homogenen Immunoassays ist, dass ein höherer chemisch-synthetischer Aufwand nötig ist, um die Bindungsreaktion mit der Signalgenerierung zu koppeln.

Im Patent US4960693 A wird die Synthese eines Antikörper-Enzymfragment-1-Konjugates beschrieben, so dass erst nach der Bindung des Antigen-Enzymfragment-2-Konjugats das funktionsfähige Enzym ("Holoenzym") entsteht. Die chemische Synthese solcher Konjugate ist sterisch anspruchsvoll und nicht für alle Arten von Analyten gleich gut geeignet. Daneben wird durch die Kopplung mit der Enzymreaktion eine zusätzliche Reaktionszeit nötig, so dass dieses Prinzip sich nicht durchsetzen konnte.

Ein universellerer und chemisch einfacherer Ansatz für niedermolekulare Analyten ist der Fluoreszenzpolarisations-Immunoassay, wie z.B. in EP0200960A beschrieben. Dabei wird ein Konjugat aus dem Analyten (hier: Östriol) und einem Fluoreszenzfarbstoff, meist Fluorescein, synthetisiert. Die Messlösung wird mit linear polarisiertem Anregungslicht beleuchtet. Das emittierte Fluoreszenzlicht ist nicht polarisiert (depolarisiert), solange das Konjugat frei in Lösung vorliegt. Erst nach Bindung an den Antikörper wird die Rotationsgeschwindigkeit des Konjugates so weit eingeschränkt, dass auch das emittierte Fluoreszenzlicht polarisiert ist. Damit kann das Bindungsgleichgewicht in Echtzeit gemessen werden. Der Nachteil dieses Verfahrens ist der hohe apparative Aufwand für die Detektion, da eine polarisierte monochromatische Lichtquelle und zwei mit Polarisationsfiltern ausgerüstete Fluoreszenzdetektoren für die Detektion benötigt werden. Dieses Prinzip konnte sich daher nur bei speziellen Laboranwendungen, nicht jedoch bei Routinediagnostik und im Vor-Ort-Einsatz durchsetzen. Außerdem ist es nur für niedermolekulare Analyten geeignet. Für Protein-Analyten wird ein zusätzliches Verfahren benötigt.

Eine technisch einfachere Detektion niedermolekularer Analyten wurde in dem Verfahren von Sellrie et al. am Beispiel eines Europium-Kryptat-Immunoassays (Abk: EuKr) realisiert [US2008199972A]. Hier wird ein EuKr-Fluorescein-Konjugat synthetisiert, wobei ein möglichst kurzer Linker, der aus einer bis höchstens drei Methylengruppen besteht, zwischen dem EuKr und dem Fluorescein sein darf. Neben dem Anti-EuKr-Antikörper wird zusätzlich ein Anti-Fluorescein-Antikörper, der nach Bindung an Fluorescein, dessen Fluoreszenz löscht, eingesetzt. Das Signalgenerierungsprinzip beruht darauf, dass aus sterischen Gründen nur einer der beiden Antikörper das Konjugat binden kann. Das Bindungsgleichgewicht und damit die Fluoreszenzintensität ist von der Konzentration an freiem EuKr abhängig und kann direkt in Echtzeit gemessen werden. Vorteil dieses Systems ist, dass nur ein herkömmlicher Fluoreszenzdetektor benötigt wird. Der Nachteil ist, dass es ebenfalls nur für niedermolekulare Analyten geeignet ist, und dass die Konjugatsynthese chemisch anspruchsvoll ist.

Ein alternatives homogenes Immunoassayverfahren für niedermolekulare Analyten, das ebenfalls auf dem Prinzip der bindungsabhängigen Fluoreszenzlöschung beruht, wurde von Coille et al. und, in modifizierter Form von Tan et al. publiziert [I. Coille, S. Reder, S. Bucher and G. Gauglitz, Biomol. Eng 18 (2002) 273-280,/ Chongxiao Tan, Nenad Gajovic-Eichelmann, Walter F.M. Stöcklein, Rainer Polzius, Frank F. Bier, Analytica Chimica Acta 658 (2010) 187-192]. Hier wird der Analyt, Tetrahydrocannabinol, an ein Protein, Rinderserumalbumin, gekoppelt, das an der Oberfläche zusätzlich mehrere Fluoreszenzquencher-Moleküle trägt. Der Anti-Tetrahydrocannabinol-Antikörper ist mit einem Fluroeszenzmolekül konjugiert. Die Fluoreszenz wird gelöscht, wenn der Antikörper an das Konjugat bindet. Enthält die Analysenprobe freies Tetrahydrocannabinol, bindet der Antikörper dieses, und fluoresziert wieder. Der Vorteil dieses Verfahrens ist, ebenso wie bei Sellrie et al., der einfache Messaufbau für die Fluoreszenzmessung genutzt werden kann. Nachteilig ist auch hier die chemisch anspruchsvolle Synthese des Analyt-Quencher-Konjugates und des Antikörper-Fluorophor-Konjugates.

Obwohl die Fluoreszenzmesstechnik in den Biowissenschaften sehr oft eingesetzt wird, ist sie eine aufwendige und damit teure Messtechnik. Preiswerte und kompakte Detektoren, z.B. für den Vor-Ort-Einsatz, werden eher mit anderen Nachweisverfahren realisiert. Elektrochemische Detektionsverfahren ermöglichen die technisch einfachsten und am höchsten integrierten Messgeräte und haben sich z.B. im Bereich der Einweg-Biosensoren für Glucose gegenüber allen optischen Messverfahren durchgesetzt. Es gibt daher seit vielen Jahren Bemühungen, homogene Immunoassays auf der Basis eines einfachen elektrochemischen Detektionsprinzips zu realisieren.

Voraussetzung für einen homogenen Immunoassay mit elektrochemischer Detektion ist die Verfügbarkeit einer sensitiven elektroanalytischen Methode und eines redoxaktiven Labels/Markers mit hoher spezifischer Aktivität. Amperometrische und voltammetrische Verfahren sind sensitive und geeignete Verfahren. Die spezifische Aktivität des Redoxlabels/markers hängt vor allem von der Geschwindigkeitskonstante des heterogenen Elektronentransfers mit der Elektrode und vom Detektionspotential ab. Es herrscht eine übereinstimmende Ansicht unter Fachleuten, dass ein Detektionspotential im Bereich von -200 mV bis +200 mV (gegen Silber/Silberchlorid-Referenzelektrode, künftig mit vs. Ag/AgCl abgekürzt) für Messungen in biologischen Lösungen wie Blut optimal ist. Die Elektronentransferkonstante ist eine Funktion der chemischen Struktur des Redoxlabels/markers, sowie des Diffusionskoeffizienten und des verwendeten Elektrodenmaterials. Eine Vielzahl von Redoxmediatoren sind dem Fachmann bekannt, die günstige elektrochemische Eigenschaften haben und damit prinzipiell geeignete Label/Marker für einen Immunoassay sind. Für den Einsatz in biologischen Medien dürfen die Redoxmediatoren nicht mit Probenbestandteilen reagieren, müssen im oxidierten und reduzierten Zustand stabil und ausreichend wasserlöslich sein. Dazu gehören z.B. die organischen Moleküle wie Hydrochinon/Chinon, p-Aminophenol, organisch/anorganische Sandwichmoleküle wie Ferrocen und anorganische Komplexe wie z.B. Hexacyanoferrat (II/III) oder Osmium-di-Bipyridin. Die Wasserlöslichkeit von Ferrocen und Osmium-di-Bipyridin ist schlecht, so dass hier meist wasserlösliche Derivate zum Einsatz kommen.

US2007/054317 beschreibt wasserlösliche Osmium-basierte Redoxmoleküle mit den oben genannten günstigen Eigenschaften und verschiedene elektrochemische Immunassayformate und Elektrodengeometrien, z.B. Interdigitalelektroden, mit denen die genannten wasserlöslichen Redoxmoleküle sensitiv detektiert werden können. Die in US2007/054317 beschriebenen Assayformate gehen außer durch die Verwendung der neuen Redoxmediatoren und darauf beruhender Antikörper- und Antigen-Konjugate nicht über den Stand der Technik hinaus, insbesondere wird kein neues, hoch sensitives homogenes Immunoassayformat beschrieben.

EP1331482A1 beschreibt ein elektrochemisches Immunoassayverfahren, wobei Konjugate aus Ferrocen und anderen Redoxmediatoren mit dem Analyten eingesetzt werden. Zur Detektion wird ein Enzymbiosensor genutzt, z.B. ein Glucosesensor, indem die Freisetzung des Ferrocen-Analyt-Konjugates zu einer Modulation des elektrochemischen Signals des Glucosesensors führt. Ein Nachteil der beschriebenen Kopplungschemie ist, dass ein Protein wie Humanserumalbumin benutzt wird, um ein gut wasserlösliches Konjugat herzustellen. Dem Fachmann ist bekannt, dass es nicht möglich ist, Protein-Redoxmediator-Konjugate mit einem genau definierten stöchiometrischen Verhältnis zu synthetisieren. Damit ist die Charakteristik des Tests von Charge zu Charge stets verschieden. Dem Fachmann ist auch bewusst, dass ein solch indirektes Signalverfahren die Gefahr in sich birgt, dass Hemmstoffe aus der Probe die Enzymreaktion ebenso modulieren können wie das Redoxmediator-Analyt-Konjugat. Robuster wäre ein Verfahren, bei dem die das Redoxmediatorkonjugat direkt das elektrochemische Signal moduliert.

Nach einem solchen Prinzip ist der homogene elektrochemische Assay für Hippursäure in einem Mikrofluidikchip von Sung et al. aufgebaut [Sung Ju Yoo, Young Bong Choi, Jong Il Ju, Gun-Sik Tae, Hyug Han Kim and Sang-Hoon Lee. Analyst 134 (2009), 2462-2467]. Es wird ein Ferrocen-Hippursäure-Konjugat synthetisiert und ein Anti-Hippursäure-Antikörper genutzt. Konjugat und der an Polymerpartikel gebundene Antikörper werden in genau definierter Menge der Probe zugesetzt. Enthält eine Probe keine freie Hippursäure, bindet das Konjugat an die Antikörper-beladenen Partikel und wird mit diesen abzentrifugiert. Im Überstand wird in einem voltammetrischen Experiment wird beim Oxidationspotential des Ferrocens ein entsprechend kleiner Strom fließen. Enthält die Probe dagegen freie Hippursäure, so bindet der partikelgebundene Antikörper bevorzugt daran, das Konjugat bleibt beim Zentrifugieren in Lösung und es wird im voltammetrischen Experiment ein ensprechend größerer Strom fließen (beim Oxidationspotential des Ferrocens). Dieser Assay und alle, die diesem Prinzip folgen, haben schwerwiegende Nachteile, die einen Einsatz in der Diagnostik praktisch nicht zulassen.

Erstens ist das Oxidationspotential von Ferrocen (+400 mV vs. Ag/AgCl) zu hoch für die selektive Detektion in Blut und Blutserum. Zweitens ist ein Zentrifugationsschritt für die meisten Vor-Ort-Anwendungen nicht akzeptabel. Drittens ist dieses Verfahren trotz des Separationsschrittes (Zentrifugation) sehr unempfindlich. So haben Sung et al. als kleinste Konzentration ca. 10 mg/mL Hippursäure gemessen, das entspricht einer Konzentration von 55 mM. Typische Immunoassay-Analyten müssen jedoch im nanomolaren Konzentrationsbereich (oder darunter) gemessen werden, d.h. eine Million mal empfindlicher.

Ein homogener elektrochemischer Assay, der ohne Separationsschritt auskommt, wurde von Di Gleria et al. [Katalin Di Gleria, H. Allen, 0. Hill, Calum J. McNeil, Anal. Chem. 1988, 58, 1203-1205] vorgestellt. Auch hier wird ein Konjugat aus Ferrocen und dem Analyten, Lidocain, sowie der Anti-Analyt-Antikörper der Probe zugesetzt. Als Detektionsprinzip wird eine Enzymreaktion genutzt, für die Ferrocenium-Antigen-Konjugat als Redoxmediator wirkt. Enthält die Probe keinen freien Analyten, bindet das Konjugat an den Antikörper und die Enzymreaktion, hier Glucoseoxidase, wird verzögert, der amperometrische Strom wird geringer. Der Nachteil dieses Formats ist, dass es nicht sensitiv genug für einen typischen Immunoassay ist. So wurde in Serum eine untere Nachweisgrenze von ca. 5 µM erzielt, ca. 1000-fach höher als in typischen Immunoassays.

Eine Variante dieses homogenen Redoximmunoassays wurde beschrieben, bei der der Redoxmediator-Konjugat direkt elektrochemisch gemessen wird. Auch hier wurden nur unzureichende Nachweisgrenzen erzielt.

Dieser Assay und alle, die einem ähnlichen Prinzip folgen (d.h. der Modulation der Diffusionskonstante durch die Antikörperbindung), haben schwerwiegende Nachteile, die einen Einsatz in der Diagnostik erschweren. Der Hauptnachteil ist die niedrige Sensitivität, da die Modulation der Diffusionskonstante nur eine geringe Signalmodulation nach sich zieht: Auch wenn das redoxaktive Konjugat komplett vom Antikörper gebunden wird, kann man noch ein nennenswertes amperometrisches oder voltammetrisches Signal messen. Wünschenswert wäre es, wenn das antikörpergebundene Konjugat gar kein elektrochemisches Signal produzieren würde.

Gleiches gilt für das Prinzip der Modulation einer Enzymreaktion durch die Verknappung des Redoxmediators nach Bindung an einen Antikörper. Auch dieses Format konnte nur mikromolare Konzentrationen (oder höher) detektieren.

Insgesamt kann man sagen, dass homogene elektrochemische Immunoassays, die auf der Modulation der Diffusionskonstante eines niedermolekularen Konjugates oder der Modulation einer Enzymreaktion durch die Verknappung des Redoxmediators nach Bindung eines Antikörpers beruhen, nicht ausreichend sensitiv sind, um Konzentrationen im nanomolaren Bereich (oder darunter) zu messen, wie sie für Immunoassay-Analyten typisch sind. Deshalb konnte trotz des preiswerten Detektionssystems keiner dieser Assays eine nennenswerte kommerzielle Anwendung finden.

Wei et al. beschreiben in Biosensors and Bioelectronics, Elsevier Advanced Technology, Bd. 24., Nr. 9, S. 2909-2914 (2009), die Entwicklung eines redoxmarkierten elektrochemischen Immunoassays.

Ghindils et al. in Analytical Letters, Bd. 28, Nr. 1. S. 1-11 (1995), und Sapin et al. in Clinical Chemistry and Laboratory Medicine, Bd. 45, Nr. 3, S. 416-418 (2007) offenbaren jeweils Redoxmarker-Antikörper, die spezifisch an einen Redoxmarker (Ferrocen bzw. Ruthenium) binden und die Redoxaktivität des daran gebundenen Redoxmarkers weitgehend unterbinden.

US 2004/0020791 A1 beschreibt einen heterogenen Immunoassay unter Verwendung von Redoxmarker-Antikörpern, bei dem in einer Ausführungsform ein bispezifisches Antikörperkonjugat, das an einen auf einer festen Phase immobilisierten Redoxmarker bindet, zur Detektion verwendet wird.

Forrow et al. offenbaren in Bioconjugate Chemistry, Bd. 15, Nr. 1, S. 137-144 (2004), ein Verfahren zur Durchführung von homogenen Immunoassays mit elektrochemischer Detektion in Lösung, jedoch ohne Verwendung eines bispezifischen Antikörperkonjugats.

Vor diesem Hintergrund liegt die Aufgabe der vorliegenden Erfindung in der Bereitstellung eines neuen verbesserten Verfahrens zur Durchführung eines Immmunoassays in Lösung, welches die genannten Nachteile des Standes der Technik nicht mehr aufweist und insbesondere eine deutlich verbesserte Sensitivität bietet.

Diese Aufgabe wird erfindungsgemäß gelöst mit dem Verfahren nach Anspruch 1. Speziellere Ausführungsformen und weitere Aspekte der Erfindung sind Gegenstand der weiteren Ansprüche.

Das erfindungsgemäße Verfahren zur Durchführung von homogenen kompetitiven Immunoassay-Formaten mit elektrochemischer Detektion ist dadurch gekennzeichnet, dass zwei verschiedene Konjugate als Reagenzien mit der Probe oder dem Proben/Puffer-Gemisch kombiniert werden, wobei das eine Konjugat einen Redoxmarker und ein Analytmolekül umfasst und das zweite Konjugat einen Anti-Redoxmarker-Antikörper oder ein spezifisch bindendes Fragment davon und ein den Analyten spezifisch bindendes Molekül umfasst.

Der Analyt kann grundsätzlich jedes in einem Immunoassay oder anderen Bindungsassay nachweisbare Molekül sein, für das ein spezifischer Bindungspartner existiert. Der Analyt kann niedermolekular sein, d.h. eine Molmasse von ≤ etwa 1000 Dalton aufweisen, oder höher- und hochmolekular sein. Beispiele für höher- und hochmolekulare Analyten sind Nukleinsäuren, Peptide und Proteine. Besonders bevorzugte Analyten sind niedermolekulare organische Moleküle und Proteine/Peptide, z.B. Hormone, Enzyme, Biomarker, biologische Wirk- und Schadstoffe und andere physiologisch oder metabolisch relevante Substanzen.

Das den Analyten spezifisch bindende Molekül kann ein Anti-Analyt-Antikörper oder ein Fragment davon, ein Aptamer, ein Peptid, eine Nukleinsäure oder ein, typischerweise organischer, Chelator, der den Analyten spezifisch (z.B. in einer speziellen Bindungstasche oder Koordinationsstruktur) bindet und kein Antikörper ist (in der Literatur auch als "Wirtsmolekül" für ein "Gastmolekül" bezeichnet), sein. Vorzugsweise handelt es sich um einen Anti-Analyt-Antikörper oder ein Fragment davon.

Ferner ist das erfindungsgemäße Verfahren dadurch gekennzeichnet, dass die Gegenwart von freiem Analyten in der Probe die Bindung des Redoxmarkers an den Anti-Redoxmarker-Antikörper oder das spezifisch bindende Fragment davon veranlasst oder fördert, wobei durch diese Bindung die Redoxaktivität des gebundenen Redoxmarkers unterbunden ("gelöscht" oder "gequencht") wird, wodurch ein detektierbares Signal erzeugt oder verändert wird. Das erfindungsgemäße Verfahren ist oder beinhaltet einen kompetitiven Assay.

Eine geeignete Verfahrensgestaltung ist beispielsweise wie folgt: Ein bispezifisches Antikörperkonjugat, umfassend einen Anti-Analyt- und einen Anti-Redoxmarker-Antikörper, und das Redoxmarker-Analyt-Konjugat, werden in Puffer gelöst. Ein Teil der Konjugat-Moleküle bindet an das bispezifische Antikörper-Konjugat, die Mehrzahl davon am Anti-Analyt-Ende des bispezifischen Antikörperkonjugates, da der Anti-Analyt-Antikörper, insbesondere unter den sterischen Bedingungen des Konjugats, vorzugsweise eine deutlich höhere Affinitätskonstante als die des Anti-Redoxmarker-Antikörpers besitzt. Das Redoxmarker-Ende des Konjugates wird daher praktisch nicht gebunden und ist so der elektrochemischen Messung (z.B. Amperometrie oder Voltammetrie) frei zugänglich und es wird ein hoher Reduktionsstrom gemessen. Kommen nun freie Analytmoleküle aus der Probe dazu, verdrängen diese das Analytende des Analyt-Redoxmarker-Konjugates aus den Bindungsstellen des Analyt-Antikörpers. Erst jetzt können die Redoxmarker-Enden des Konjugates an den Anti-Redoxmarker-Antikörper-Teil des bispezifischen Antikörperkonjugates binden, wodurch die Redoxaktivität des gebundenen Redoxmarkers unterbunden ("gelöscht") wird, und es wird ein verringertes elektrochemisches Signal gemessen.

Das erfindungsgemäße Verfahren geht über den Stand der Technik weit hinaus, da es erstmalig das Prinzip des "Löschens" eines Signals durch die Antikörper-Antigen-Bindung (analog dem Fluoreszenz-Löschen bzw. Fluoreszenz Quenching) auf die direkte elektrochemische Detektion überträgt. Fluoreszenz Quenching Immunoassays gehören zu den sensitivsten und schnellsten homogenen Immunoassays, da das Signal in Echtzeit proportional zur Antikörper-Antigen-Bindung generiert wird. Eine solche Signalcharakteristik wurde nun erstmals auch bei einer elektrochemischen Detektion realisiert.

Als redoxaktive Marker (in Analogie zum Fluorophor) können erfindungsgemäß Ferrocen (bzw. beliebige Derivate des Ferrocens), Osmium-di-Bipyridyl-Komplexe und andere Osmium-basierte Komplexe, Ruthenium-Bipyridyl-Komplexe und andere Ruthenium-basierte Komplexe, p-Aminophenol, Hexacyanoferrat (II/III), Hydrochinone/Chinone, oder andere Redoxmediatoren, insbesondere andere für Immunoassays bekannte und geeignete Redoxmarker, verwendet werden. Die "Löschung" der Redoxaktivität des Ferrocens, oder eines anderen Redoxmediators, war bisher im Stand der Technik unter den Bedingungen eines Immunoassays nicht möglich. Zwar wurde in Arbeiten wie der von Nielson et al. [Roger M. Nielson, Joseph T. Hupp, Inorg. Chem. 1996, 35, 1402-1404] beschrieben, dass Calixarene, die eine schwache Bindung zu Ferrocen ausbilden, in der Lage sind, die Redoxaktivität von Ferrocenen komplett zu löschen, jedoch erst bei Konzentrationen von ca. 10 mM und höher. So hohe Konzentrationen eines Redox-Quenchers sind für Immunoassays völlig ungeeignet.

Überraschender Weise wurde gefunden, dass monoklonale Antikörper, die von einem der Erfinder zur Bindung von Ferrocen generiert wurden, eine solche Löschungs-Charakteristik aufweisen. Ein besonders wirkungsvoller neuer monoklonaler Antikörper bindet ausschließlich die oxidierte Form von Ferrocen, das Ferrocenium-Kation (oder Ferricinium), künftig Ferrocenium genannt. Die Redoxaktivität des gebundenen Ferroceniums wird dadurch komplett gelöscht, es lässt sich im gebundenen Zustand nicht mehr an einer Elektrode (Glaskarbon, Edelmetall) reduzieren. Der Ferrocenium-bindende Antikörper wird in den Ausführungsbeispielen des erfindungsgemäßen Verfahren als Redox-Quencher (in Analogie zum Fluoreszenz Quencher) genutzt.

Im Rahmen des erfindungsgemäßen Verfahrens kann jedoch auch jeder andere Antikörper genutzt werden, der den im Test benutzten Redoxmediator bindet und dadurch dessen Redoxaktivität unterbindet (löscht). Es ist dabei für die Erfindung unerheblich, ob der Antikörper den oxidierten oder den reduzierten Zustand des Redoxmediators bindet. Besondere Vorteile bietet in manchen Anwendungen jedoch ein Antikörper, der den oxidierten Mediator bindet, wie im Falle des Anti-Ferrocenium-Antikörpers, der in der beschriebenen Erfindung erstmals angewendet wird. Der Grund ist das niedrigere Detektionspotential, das für die Reduktion des Redoxmarkers genutzt werden kann (im Anwendungsbeispiel wird bei +100 mV vs. Ag/AgCl detektiert, im Falle der Oxidation müsste bei +250 bis +400 mV detektiert werden, was erhöhte unspezifische Signale in Blutserum zur Folge hätte.)

Diese Redoxmarker-Antikörper mit Quencher-Wirkung und diese umfassende bispezifische Antikörperkonjugate werden in der vorliegenden Erfindung verwendet. Die erfindungsgemäß verwendeten Redoxmarker-Antikörper mit Quencher-Wirkung sind in der Lage, die Redoxaktivität des gebundenen Redoxmarkers weitgehend, vorzugsweise zu mehr als 80 %, bevorzugter mehr als 90 %, noch bevorzugter mehr als 95 %, oder vollständig zu unterbinden (zu "löschen" oder "quenchen").

Der Begriff "Antikörper", wie in der vorliegenden Anmeldung verwendet, soll grundsätzlich auch spezifisch bindende Fragmente davon umfassen, soweit aus dem Zusammenhang nicht eindeutig etwas Anderes hervorgeht.

Mit einem hier erstmals beschriebenen Anti-Ferrocenium-Antikörper läßt sich ein kompetitiver Immunoassay zur Detektion von Ferrocenium (oder Ferrocen) realisieren, der jedoch keine große technische Bedeutung hätte. Einen hohen technischen Nutzen und einen hohen kommerziellen Wert hat das Verfahren erst, wenn sich damit beliebige Analyten mit dem elektrochemischen Immunoassay nachweisen lassen. Zu diesem Zwecke muss die Ferrocenium-Anti-Ferrocenium-Immunkomplexbildung mit einem beliebigen monoklonalen Antikörper gekoppelt werden können, derart, dass die Bindung des Zielanalyten an den komplementären Antikörper eine Modulation der Ferrocenium-Anti-Ferrocenium-Bindung zur Folge hat. Der von Sellrie et al. [US2008199972A] beschriebene, elegante Ansatz der Kopplung zweier Immunreaktionen durch ein Konjugat aus beiden Antigenen, die mit einem sehr kurzen Linker verbunden sind, so dass jeweils nur der Anti-Pestizid-Antikörper oder der Anti-Fluorescein-Antikörper binden kann, ließ sich im Falle des erfindungsgemäßen Verfahrens nicht nutzen, da Ferrocenkonjugate, die mit niedermolekularen Substanzen gekoppelt werden, in der Regel nicht wasserlöslich sind. Für die praktische Nutzung musste ein Ferrocen-Analyt-Konjugat hergestellt werden, dass eine ausreichend hohe Wasserlöslichkeit aufweist. Dem Fachmann sind eine Anzahl wasserlöslicher Linkermoleküle bekannt, die für die Synthese von Biokonjugaten genutzt werden können. Polyethylenglykol-Linker (Synonym: Polyethylenoxid-Linker, PEG, PEO) gehören aufgrund der guten Wasserlöslichkeit, der geringen unspezifischen Bindung an Proteine und Oberflächen und der Verfügbarkeit einer Vielzahl von Kettenlängen und chemischen Funktionen zu den am häufigsten genutzten wasserlöslichen Linkern. Polyethylenglykol-Linker (Synonym: Polyethylenoxid-Linker) sind lineare Moleküle, die an einem oder beiden Enden koppelbare Funktionen aufweisen können, z.B. Carboxylgruppen, Aminogruppen, Thiolgruppen usw. Die Wasserlöslichkeit von Polyethylenglykol-Linkern ist sehr hoch, und sie nimmt mit zunehmender Kettenlänge des Linkers zu.

Zur Herstellung der im erfindungsgemäßen Verfahren verwendeten Konjugate wird ein bifunktioneller wasserlöslicher Linker ausreichender Kettenlänge, z.B. ein 400-30.000 Da PEG-Linker mit zwei terminalen koppelbaren Funktionen, vorzugsweise ein 2000 Da Diamino-PEG-Linker benutzt, um an einem Ende Ferrocen, oder einen anderen geeigneten Redoxmediator, und, am anderen Ende, den Zielanalyten zu koppeln. Durch die Verwendung von PEG-Linkern ausreichender Länge können auch in Wasser unlösliche Analyten dem Test zugänglich gemacht werden. Homobifunktionelle Linker, die an beiden Enden die gleiche Kopplungsfunktion aufweisen, können ebenso genutzt werden wie heterobifunktionelle Linker, die zwei unterschiedliche Kopplungsfunktionen aufweisen.

In Bindungsstudien mit dem Ferrocen-Analyt-Konjugaten in Anwesenheit des freien Anti-Ferrocenium-Antikörpers, des freien Anti-Analyt-Antikörpers und des Ferrocen-Analyt-Konjugates zeigte sich, dass keine ausreichende Modulation des Ferrocen-Signals durch den Analyten erfolgte. Es zeigte sich auch, dass das Ferrocenium-Analyt-Konjugat über die gesamte Analysendauer im oxidierten Zustand gehalten werden musste, um messbare Signale zu erzielen. Überraschend wurde herausgefunden, dass in einigen sauren Puffern Ferrocen dauerhaft im oxidierten Zustand bleibt. Im erfindungsgemäßen Verfahren wird der Assay vorzugsweise bei pH-Werten zwischen pH 1 und pH 7, besonders bevorzugt zwischen pH 4 und pH 5, durchgeführt. Geeignete Puffersalze sind z.B. Phosphat, MES, HEPES, TRIS, Bistris, Acetat, Glycylglycin, Glycin.

Es wurde nun ein überraschend einfaches, neues Prinzip gefunden, um die Ferrocenium-Anti-Ferrocenium-Bindungsreaktion mit der Analyt-Anti-Analyt-Bindungsreaktion zu koppeln. Dazu wurde ein bispezifisches Antikörperkonjugat hergestellt, indem der Anti-Ferrocenium-Antikörper im stöchiometrischen Verhältnis (1:1) mit dem Anti-Analyt-Antikörper gekoppelt wurde, wobei ein kurzer Linker von höchstens 10 Methyleneinheiten Länge verwendet wurde. Damit konnte erstmals die oben beschriebene, gewünschte Signal-Löschungscharakteristik in einem elektrochemischen Immunoassay erzielt werden.

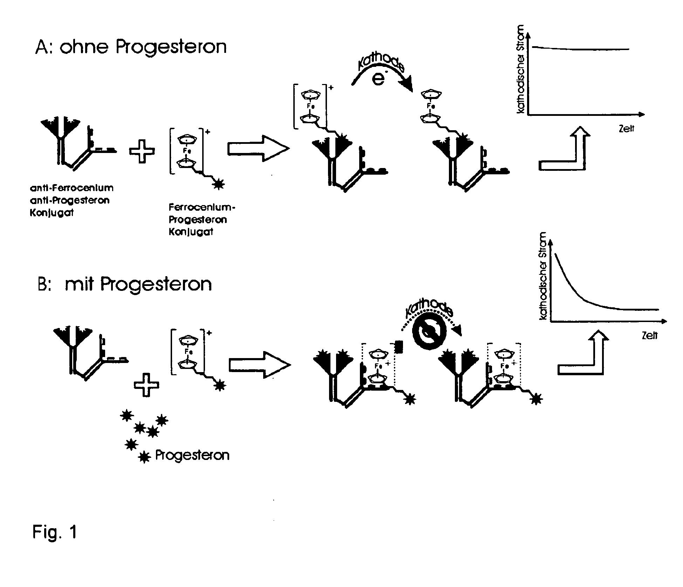

Das Funktionsprinzip, erklärt am Beispiel eines kompetitiven Progesteron-Immunoassays, ist dabei wie folgt: Das bispezifische Antikörperkonjugat, bestehend aus einem Anti-Progesteron- und einem Anti-Ferrocenium-Antikörper, und das Ferrocenium-Progesteron-Konjugat werden in Puffer gelöst. Ein Teil der Konjugat-Moleküle bindet an das bispezifische Antikörper-Konjugat, die Mehrzahl davon am Anti-ProgesteronEnde des bispezifischen Antikörperkonjugates (da dieser Antikörper eine höhere Affinitätskonstante besitzt). Das Ferrocenium-Ende des Konjugates wird praktisch nicht gebunden, da der PEG-Linker dafür zu kurz ist, und außerdem eine energetisch ungünstige, gebogene Konformation annehmen müsste. Das Ferrocenium-Ende ist daher der elektrochemischen Messung frei zugänglich (Amperometrie oder Voltammetrie) und es wird ein hoher Reduktionsstrom gemessen. Kommen nun freie Progesteronmoleküle aus der Probe dazu, verdrängen diese das Progesteronende des Progesteron-Ferrocenium-Konjugates aus den Bindungsstellen des Progesteron-Antikörpers. Erst jetzt können die Ferrocenium-Enden des Konjugates an den Anti-Ferrocenium-Antikörper-Teil des bispezifischen Antikörperkonjugates binden und es wird ein verringertes elektrochemisches Signal gemessen (Veranschaulichung siehe Fig. 1).

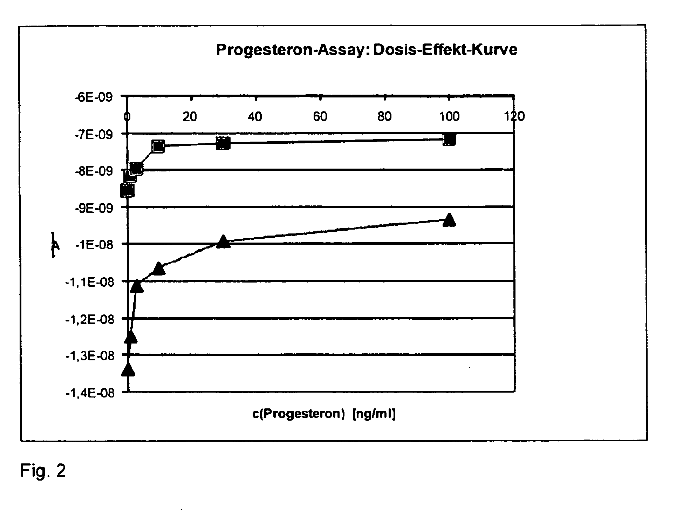

Bei Zugabe eines Überschusses an bispezifischem Antikörperkonjugat im Vergleich zum Ferrocenium-Analyt-Konjugat werden die meisten Ferrocenium-Reste vom Antikörperkonjugat gebunden und das elektrochemische Signal wird fast auf null reduziert, also gelöscht. Es ist dem Fachmann jedoch bekannt, dass die Antikörperkonzentration bzw. die Konzentration des bispezifischen Antikörpers im Immunoassay so gewählt wird, dass sich eine maximale Sensitivität für Progesteron ergibt, so dass das Antikörperkonjugat in einem hoch empfindlichen Assay im Unterschuss zugesetzt wird. In diesem Fall wird das elektrochemische (Gesamt-)Signal auch bei hoher Progesteronkonzentration nicht komplett gelöscht. Es hat sich gezeigt, dass ein Progesteron-Assay am sensitivsten ist, wenn das elektrochemische Signal durch Progesteronzugabe nur zu ca. 50 % gelöscht werden kann (siehe Fig. 2).

Die erzielte Signal-Löschungs-Charakteristik wurde bisher noch nicht bei elektrochemischen Immunoassays gezeigt, weil keine entsprechenden Quencher-Moleküle zur Verfügung standen.

Die erfindungsgemäß genutzten Anti-Ferrocenium-Antikörper sind ein Beispiel für ein solches Redox-Quencher-Molekül. Es ist jedoch ebenso möglich, analoge Antikörper herzustellen, die andere Redoxmarker binden und deren Signal löschen. Der Einsatz solcher Antikörper in einem bispezifischen-Antikörperkonjugat ist ebenfalls Teil der Erfindung.

Die vorliegende Erfindung stellt ein Verfahren zur Durchführung von direkten, homogenen Immunoassays in Lösung mit sensitiver elektrochemischer Detektion bereit, das dadurch gekennzeichnet ist, dass nanomolare Konzentrationen von niedermolekularen oder hochmolekularen Analyten gemessen werden können, dass keines der Reagenzien immobilisiert ist, keine Separations- und Waschschritte erfolgen und die Bindungsreaktion in Echtzeit elektrochemisch detektiert wird.

Die technische Anwendung des erfindungsgemäßen Verfahrens ist besonders einfach, da es mit verschiedenen elektroanalytischen Techniken genutzt werden kann, die dem Fachmann bekannt sind, keinerlei Modifikation der Messelektrode verlangt (z.B. chemische Modifikation, Membranen, Beschichtung mit Dünnen Filmen, laterale Strukturierung etc.) und praktisch beliebig skalierbar ist (Verringerung der Elektrodendimensionen, Verringerung des Analysenvolumens). Da das Signalgenerierungsprinzip, d.h. die Löschung der Redoxaktivität eines beliebigen Redoxmediators, sich auf alle bekannten elektroanalytischen Messtechniken, wie z.B. Amperometrie, Voltammetrie, Coulometrie, Amperometrische Titration, Coulometrische Titration, Potentiometrie, Impedanzspektroskopie usw. anwenden lässt, kann das erfindungsgemäße Verfahren mit allen diesen Verfahren zu neuen Immunoassay-Anwendungen kombiniert werden. So kann in manchen Fällen Amperometrie die Methode der Wahl sein, z.B. um ein möglichst preiswertes Gesamtsystem zu erzielen, oder um bestehende amperometrische Detektoren (z.B. Glucose-Biosensor-Messgeräte) für den Einsatz von Immunoassays einzusetzen. Je nach Anwendungsfall und gewünschtem Analyt ist es möglich, die Elektroanalysen-Technik zu variieren, ohne den Immunoassay grundsätzlich neu entwickeln zu müssen.

Die Variabilität und technische Anwendbarkeit des neuen Immunoassay-Prinzips zeigt sich besonders deutlich, wenn es in mikrofluidischen Analysesystemen (Lab-on-a-Chip Systemen) genutzt wird. Dies ist besonders leicht möglich, da es sich um einen homogenen Immunoassay, ohne immobilisierte Komponenten und ohne Separationsschritte handelt. Die Reagenzien können in flüssiger oder getrockneter Form in das Lab-on-a-Chip System eingebracht werden, so dass sie durch Zugabe der Probe freigesetzt werden und der Assay gestartet wird. Das Messsignal kann kontinuierlich gemessen werden, oder nur zu Beginn und zum Ende der Analyse (nach Erreichen des Bindungsgleichgewichtes). Es ist keinerlei Synchronisation von Abläufen, Dosier- und Wasch- oder Zentrifugationsschritten nötig, um das Analysenergebnis zu bekommen. Im Gegensatz zu vielen optischen Messverfahren (z.B. Photometrie, Fluoreszenzmessung) verschlechtert sich das Signal-zu-Rausch-Vehältnis von elektroanalytischen Verfahren auch bei Miniaturisierung nicht, d.h. es müssen für miniaturisierte Elektroden keine neuen und aufwendigen Detektoren und Verstärker entwickelt werden.

Der hohe Wert des neuen Verfahrens zeigt sich auch darin, dass es damit möglich ist, hoch sensitive Wegwerf-Immunosensoren für den Einmalgebrauch zu realisieren, wie z.B. beim heute üblichen Schwangerschaftstest nach dem Lateral Flow Immunoassayprinzip. Die für die Detektion benötigte Elektronik (z.B. ein amperometrischer Schaltkreis) kann in einem preiwerten portablen Gerät integriert (z.B. einem Glucose-Teststreifen-Messgerät) oder sogar als Wegwerf-Elektronik realisiert sein (z.B. eine gedruckte Schaltung oder ein hoch integrierter Schaltkreis).

Ebenso ist das neue Verfahren auch für Laboranalysatoren geeignet, die voll automatisch eine Anzahl verschiedener Immunoassays parallel durchführen. Hier kommt die kurze Analysezeit des erfindungsgemäßen Verfahrens von 1 bis 5 Minuten zum Tragen.

In einer speziellen Ausführungsform des erfindungsgemäßen Verfahrens werden die benötigten Reagenzien in löslicher Form zugegeben und eine elektrochemische Detektion des Bindungsereignisses erfolgt in Echtzeit.

In einer anderen speziellen Ausführungsform werden alle benötigten Reagenzien als fertiges Gemisch zur Probe gegeben und eine elektrochemische Detektion des Bindungsereignisses erfolgt in Echtzeit (Mix-and-measure Prinzip).

In einer weiteren speziellen Ausführungsform werden alle benötigten Reagenzien als Trockenreagenzien in einem geeigneten Reaktionsgefäß (z.B. Eppendorf-Tube, Mikrotiterplatte, Lab-on-a-Chip-System, Teststreifen) zur Verfügung gestellt und erst durch Zugabe der Probe und ggf. Puffer aufgelöst, wodurch der Beginn der Analyse gekennzeichnet ist.

In einer noch weiteren, besonders günstigen speziellen Ausführungsform wird der Immunoassay in einem mikrofluidischen Analysensystem (Lab-on-a-Chip) durchgeführt.

In einer typischen Ausführungsform wird der Immunoassay auf einem voll automatischen Analysator durchgeführt.

Eine weitere Verbesserung des erfindungsgemäßen Verfahrens kann dadurch erreicht werden, dass anstelle einer einfachen Elektrode zur Detektion eine strukturierte Elektrode z.B. eine Interdigitalelektrode, verwendet wird, mit der sich die Sensitivität der Analyse durch sog. Redox-Recycling weiter erhöhen lässt.

Eine weitere vorteilhafte Modifizierung des erfindungsgemäßen Verfahrens besteht darin, dass anstelle einer einfachen elektrochemischen Redoxreaktion (z.B. Reduktion des Ferroceniums zu Ferrocen) eine enzymatisch katalysierte zyklische Reaktion genutzt wird. (z.B. elektrochemische Reduktion gefolgt von enzymatischer Reoxidation von Ferrocenium), ein sog. Enzym-Recycling, mit der sich die Sensitivität der Analyse weiter erhöhen lässt.

Das erfindungsgemäße Verfahren ist insbesondere für Messungen im niedrigen nanomolaren Konzentrationsbereich geeignet und ca. 1000-fach sensitiver als die bisher bekannten homogenen elektrochemischen Immunoassays ohne Trennschritte. Diese enorme Verbesserung der Sensitivität wird durch das oben beschriebene, neue Signalprinzip erreicht, bei dem die Redoxaktivität des redoxaktiven Konjugates nach Bindung an den Antikörper komplett gelöscht werden kann (anstatt nur die Diffusionskonstante zu verringern wie bei den bisher bekannten Verfahren).

Das elektrochemische Messverfahren ist dabei technisch ebenso einfach wie bei den oben genannten Verfahren, da eine einfache amperometrische oder voltammetrische Messung an herkömmlichen, unbeschichteten Elektroden (z.B. Glaskarbonelektroden) erfolgt. Die manuellen Handhabungsschritte beschränken sich auf die Zugabe der Reagenzien, sowie die Zugabe der Probenlösung, ein Separationsschritt ist nicht nötig. Das erfindungsgemäße Verfahren ermöglicht auch eine Analyse nach dem Mix-and-Measure Prinzip und eignet sich im besonderen Maße für die Automatisierung. Das erfindungsgemäße Verfahren ist dabei gut skalierbar, d.h. auf miniaturisierte Volumina, z.B. bei mikrofluidischen Anwendungen (Lab-on-a-Chip) anwendbar. Das erfindungsgemäße Verfahren eignet sich aufgrund dieser Charakteristik und des niedrigen Detektionspotentials von typischer Weise +100 mV (vs. Ag/AgCl) damit für die direkte Messung von niedermolekularen Analyten wie z.B. Steroidhormonen ebenso wie von hochmolekularen Analyten wie z.B. hCG oder Proteinmarkern in komplexen biologischen Proben wie z.B. Blut.

Das erfindungsgemäße Verfahren ist damit für eine Vielzahl kommerziell bedeutender Immunoassays geeignet und in der Lage, bisherige Detektionsverfahren, die technisch aufwendig (und damit kostspielig) oder schwer zu miniaturisieren und zu automatisieren sind, zu ersetzen. Es ist geeignet für Anwendungen in der klinischen Diagnostik, Umwelt- und Lebensmittelanalytik und aufgrund der guten Miniaturisierbarkeit und technischen Einfachheit insbesondere für Anwendungen außerhalb des Labors (Vor-Ort-Anwendungen, Pointof-Care Analysen, Einweg-Sensoren, Lab-on-a-Chip Immunoassays). Das erfindungsgemäße Verfahren verfügt damit über das Potential in vielen Anwendungen herkömmliche, teure Detektionssysteme wie z.B. Fluoreszenzdetektoren oder Fluoreszenzpolarisationsdetektoren, zu ersetzen.

### Kurzbeschreibung der Figuren

Fig. 1: Schematische Darstellung des erfindungsgemäßen Immunoassays am Beispiel des Progesteron-Nachweises
Fig. 2: Dosis-Effekt Kurve für die amperometrische Messung von Progesteron nach dem erfindungsgemäßen Verfahren

Die folgenden, nicht-beschränkenden Beispiele sollen die Erfindung näher erläutern.

### BEISPIEL 1

### Direkter kompetitiver Progesteronimmunoassay in Blutserum

Geräte: Elektrochemische Rührzelle 1 mL Arbeitsvolumen, Potentiostat im amperometrischen Modus

Puffer: Acetatpuffer, pH 4,4

Probe: Humanserum unverdünnt,

Kalibrator: Humanserum unverdünnt, versetzt mit 2 ng/mL, 4 ng/mL, 10 ng/mL, 20 ng/mL, 100 ng/mL, 200 ng/mL Progesteron Verfahrensschritte:
- Befüllen der elektrochemischen Messzelle mit 450µl Acetat-Puffer pH 4,4
- Polarisieren der Arbeitselektrode auf + 100 mV (vs. Ag/AgCl)
- Zugabe von 10 µl bispezifisches Antikörper-Konjugat (Endkonzentration ca. 0,1 µg/mL)
- Zugabe von 10 µl Redoxmarker-Progesteron-Konjugat (Endkonzentration ca. 100 nM)
- Zugabe von 30 µl H₂O₂/Peroxidase (Endkonzentration 5 mM H₂O₂, 0,02 nM Peroxidase)
- Messung des Basissignals
- Zugabe von 500 µl Humanserum (oder 500 µl Kalibrator)
- Messung des Endsignals 180 sec nach Serum-Zugabe

Die Auswertung erfolgt anhand einer vorher gemessenen Dosis-Effekt-Kurve oder anhand einer 2-Punktkalibrierung.

### BEISPIEL 2

### Direkter kompetitiver Progesteronimmunoassay in Speichel

Geräte: Elektrochemische Rührzelle 1 mL Arbeitsvolumen, Potentiostat im amperometrischen Modus

Puffer: Acetatpuffer, pH 4,4

Probe: Humanspeichel 1:5 verdünnt,

Kalibrator: Humanspeichel 1:5 verdünnt, versetzt mit 2 ng/mL, 4 ng/mL, 10 ng/mL, 20 ng/mL, 100 ng/mL, 200 ng/mL Progesteron

Verfahrensschritte:
- Befüllen der elektrochemischen Messzelle mit 450µl Acetat-Puffer pH 4,4
- Polarisieren der Arbeitselektrode auf + 100 mV (vs. Ag/AgCl)
- Zugabe von 10 µl bispezifisches Antikörper-Konjugat (Endkonzentration ca. 0,1 µg/mL)
- Zugabe von 10 µl Redoxmarker-Progesteron-Konjugat (Endkonzentration ca. 100 nM)
- Zugabe von 30 µl H₂O₂/Peroxidase (Endkonzentration 5 mM H₂O₂, 0,02 nM Peroxidase)
- Messung des Basissignals
- Zugabe von 500 µl Humanspeichel 1:5 verdünnt (oder 500 µl Kalibrator)
- Messung des Endsignals 180 sec nach Speichel-Zugabe

Die Auswertung erfolgt anhand einer vorher gemessenen Dosis-Effekt-Kurve oder anhand einer 2-Punktkalibrierung.

### BEISPIEL 3

### Direkter kompetitiver Humanchoriongonadotropinimmunoassay (hCG) in Urin

Geräte: Elektrochemische Rührzelle 1 mL Arbeitsvolumen, Potentiostat im amperometrischen Modus

Puffer: Acetatpuffer, pH 4,4

Probe: Humanserum unverdünnt,

Kalibrator: Humanserum unverdünnt, versetzt mit 10 mIU/mL, 20 mIU/mL, 40 mIU/mL, 100 mIU/mL, 200 mIU/mL hCG

Verfahrenschritte:
- Befüllen der elektrochemischen Messzelle mit 450µl Acetat-Puffer pH 4,4
- Polarisieren der Arbeitselektrode auf + 100 mV (vs. Ag/AgCl)
- Zugabe von 10 µl bispezifisches Antikörper-Konjugat (Endkonzentration ca. 0,3 µg/mL)
- Zugabe von 10 µl Redoxmarker-hCG-Konjugat (Endkonzentration ca. 200 nM)
- Zugabe von 30 µl H₂O₂/Peroxidase (Endkonzentration 5 mM H₂O₂, 0,02 nM Peroxidase)
- Messung des Basissignals
- Zugabe von 500 µl Humanserum (oder 500 µl Kalibrator)
- Messung des Endsignals 180 sec nach Serum-Zugabe

Die Auswertung erfolgt anhand einer vorher gemessenen Dosis-Effekt-Kurve oder anhand einer 2-Punktkalibrierung.

## Patentansprüche

1. Verfahren zur Durchführung von homogenen kompetitiven Immunoassay-Formaten mit elektrochemischer Detektion in Lösung, **dadurch gekennzeichnet, dass** zwei verschiedene Konjugate als Reagenzien mit der Probe oder einem ProbenPuffer-Gemisch kombiniert werden, wobei das eine Konjugat einen Redoxmarker und ein Analytmolekül umfasst und das zweite Konjugat einen Anti-Redoxmarker-Antikörper oder ein spezifisch bindendes Fragment davon und ein den Analyten spezifisch bindendes Molekül umfasst, und dadurch, dass die Gegenwart von freiem Analyten in der Probe die Bindung des Redoxmarkers an den Anti-Redoxmarker-Antikörper veranlasst oder fördert, wobei durch diese Bindung die Redoxaktivität des gebundenen Redoxmarkers unterbunden ("gequencht") wird, wodurch ein detektierbares Signal erzeugt oder verändert wird.

2. Verfahren nach Anspruch 1, **dadurch gekennzeichnet, dass** der Redoxmarker aus der Gruppe aus Ferrocen und FerrocenDerivaten, Osmium-di-Bipyridyl-Komplexen und anderen Osmium-basierten Komplexen, Ruthenium-Bipyridyl-Komplexen und anderen Ruthenium-basierten Komplexen, p-Aminophenol, Hexacyanoferrat (II/III), Chinonen und anderen für elektrochemische Immunoassays bekannten Redoxmarkern ausgewählt ist.

3. Verfahren nach Anspruch 2, **dadurch gekennzeichnet, dass** der Redoxmarker Ferrocenium ist und der Anti-Redoxmarker-Antikörper ein, vorzugsweise monoklonaler, Anti-Ferrocenium-Antikörper ist.

4. Verfahren nach einem der Ansprüche 1-3, **dadurch gekennzeichnet, dass** das den Analyten spezifisch bindende Molekül einen Anti-Analyt-Antikörper, ein Aptamer, ein Peptid, eine Nukleinsäure oder einen Chelator, der den Analyten spezifisch bindet, darstellt.

5. Verfahren nach Anspruch 4, **dadurch gekennzeichnet, dass** das den Analyten spezifisch bindende Molekül ein Anti-Analyt-Antikörper oder ein Fragment davon ist.

6. Verfahren nach einem der Ansprüche 1-5, **dadurch gekennzeichnet, dass** die beiden Konjugate wasserlösliche Linkermoleküle, insbesondere PEG-Linker, umfassen.

7. Verfahren nach einem der Ansprüche 1-6, **dadurch gekennzeichnet, dass** die benötigten Reagenzien in löslicher Form zugegeben werden und eine elektrochemische Detektion des Bindungsereignisses in Echtzeit erfolgt.

8. Verfahren nach einem der Ansprüche 1-7, **dadurch gekennzeichnet, dass** alle benötigten Reagenzien als fertiges Gemisch zur Probe gegeben werden und eine elektrochemische Detektion des Bindungsereignisses in Echtzeit erfolgt.

9. Verfahren nach einem der Ansprüche 1-8, **dadurch gekennzeichnet, dass** alle benötigten Reagenzien als Trockenreagenzien in einem geeigneten Reaktionsgefäß zur Verfügung gestellt werden und durch Zugabe der Probe und ggf. Puffer aufgelöst werden, wodurch der Beginn der Analyse gekennzeichnet ist.

10. Verfahren nach einem der Ansprüche 1-9, **dadurch gekennzeichnet, dass** der Immunoassay in einem mikrofluidischen Analysensystem (Lab-on-a-Chip) und/oder auf einem vollautomatischen Analysator durchgeführt wird.

11. Verfahren nach einem der Ansprüche 1-10, **dadurch gekennzeichnet, dass** anstelle einer einfachen Elektrode zur Detektion eine strukturierte Elektrode z.B. eine Interdigitalelektrode, verwendet wird.

12. Verfahren nach einem der Ansprüche 1-11, **dadurch gekennzeichnet, dass** anstelle einer einfachen elektrochemischen Redoxreaktion eine enzymatisch katalysierte zyklische Reaktion genutzt wird.

## Claims

1. A method for performing homogeneous competitive immunoassay formats with electrochemical detection in solution, **characterized in that** two different conjugates as reagents are combined with the sample or a sample/buffer mixture, one conjugate comprising a redox marker and an analyte molecule and the second conjugate comprising an anti-redox marker antibody or a specifically binding fragment thereof and a molecule specifically binding the analyte, and **characterized in that** the presence of free analyte in the sample induces or promotes the binding of the redox marker to the anti-redox marker antibody, the redox activity of the bound redox marker being inhibited ("quenched") by this binding, whereby a detectable signal is generated or changed.

2. The method according to Claim 1, **characterized in that** the redox marker is selected from the group of ferrocene and ferrocene derivatives, bis-bipyridyl osmium complexes and other osmium-based complexes, bipyridyl ruthenium complexes and other ruthenium-based complexes, p-aminophenol, hexacyanoferrate (II/III), quinones and other redox markers known for electrochemical immunoassays.

3. The method according to Claim 2, **characterized in that** the redox marker is ferrocenium and the anti-redox marker antibody is a preferably monoclonal anti-ferrocenium antibody.

4. The method according to any one of Claims 1-3, **characterized in that** the molecule specifically binding the analyte represents an anti-analyte antibody, an aptamer, a peptide, a nucleic acid or a chelator, which specifically binds the analyte.

5. The method according to Claim 4, **characterized in that** the molecule specifically binding the analyte is an anti-analyte antibody or a fragment thereof.

6. The method according to any one of Claims 1-5, **characterized in that** the two conjugates comprise watersoluble linker molecules, in particular PEG linkers.

7. The method according to any one of Claims 1-6, **characterized in that** the required reagents are added in soluble form and an electrochemical detection of the binding event is carried out in real time.

8. The method according to any one of Claims 1-7, **characterized in that** all the required reagents are added to the sample as a ready-made mixture and an electrochemical detection of the binding event is carried out in real time.

9. The method according to any one of Claims 1-8, **characterized in that** all the required reagents are provided as dry reagents in a suitable reaction vessel and only dissolved by the addition of the sample and optionally a buffer, which marks the beginning of the analysis.

10. The method according to any one of Claims 1-9, **characterized in that** the immunoassay is performed in a microfluidic analysis system (lab-on-a-chip) and/or on a fully automated analyzer.

11. The method according to any one of Claims 1-10, **characterized in that** a structured electrode, e.g. an interdigital electrode, is used instead of a simple electrode for the detection.

12. The method according to any one of Claims 1-11, **characterized in that** an enzymatically catalyzed cyclic reaction is employed instead of a simple electrochemical redox reaction.

## Revendications

1. Procédé de réalisation de formats d'immunoanalyse compétitive homogènes avec une détection électrochimique en solution, **caractérisé en ce que** deux conjugués différents sont combinés à titre de réactifs avec l'échantillon ou un mélange d'échantillons - tampons, un conjugué comprenant un marqueur redox et une molécule d'analyte et le deuxième conjugué comprenant un anticorps anti-marqueur redox ou un fragment de celui-ci à liaison spécifique et une molécule liant spécifiquement l'analyte, et **en ce que** la présence d'analyte libre dans l'échantillon incite ou favorise la liaison du marqueur redox à l'anticorps anti-marqueur redox, l'activité redox du marqueur redox lié étant réprimée (« éteinte ») par cette liaison, moyennant quoi un signal détectable est produit ou modifié.

2. Procédé selon la revendication 1, **caractérisé en ce que** le marqueur redox est choisi dans le groupe constitué par le ferrocène ou les dérivés de ferrocène, les complexes d'osmium - di-bipyridyle et autres complexes à base d'osmium, les complexes de ruthénium - bipyridyle et autres complexes à base de ruthénium, le p-aminophénol, l'hexacyanoferrate (II/III), les quinones et autres marqueurs redox connus pour les immunoanalyses électrochimiques.

3. Procédé selon la revendication 2, **caractérisé en ce que** le marqueur redox est le ferrocénium et l'anticorps anti-marqueur redox est un anticorps anti-ferrocénium, de préférence monoclonal.

4. Procédé selon l'une des revendications 1 à 3, **caractérisé en ce que** la molécule liant spécifiquement l'analyte constitue un anticorps anti-analyte, un aptamère, un peptide, un acide nucléique ou un chélateur qui lie spécifiquement l'analyte.

5. Procédé selon la revendication 4, **caractérisé en ce que** la molécule liant spécifiquement l'analyte est un anticorps anti-analyte ou un fragment de celui-ci.

6. Procédé selon l'une des revendications 1 à 5, **caractérisé en ce que** les deux conjugués comprennent des molécules de lieurs hydrosolubles, en particulier de lieurs PEG.

7. Procédé selon l'une des revendications 1 à 6, **caractérisé en ce que** les réactifs nécessaires sont ajoutés sous une forme soluble et qu'une détection électrochimique de l'évènement de liaison se fait en temps réel.

8. Procédé selon l'une des revendications 1 à 7, **caractérisé en ce que** tous les réactifs nécessaires sont ajoutés à l'échantillon sous forme de mélange fini et qu'une détection électrochimique de l'évènement de liaison se fait en temps réel.

9. Procédé selon l'une des revendications 1 à 8, **caractérisé en ce que** tous les réactifs nécessaires sont mis à disposition sous forme de réactifs secs dans un récipient de réaction approprié et sont dissous par addition de l'échantillon et le cas échéant de tampon, moyennant quoi le début de l'analyse est caractérisé.

10. Procédé selon l'une des revendications 1 à 9, **caractérisé en ce que** l'immunoanalyse est réalisée dans un système d'analyse microfluidique (Lab-on-a-Chip) et/ou sur un analyseur entièrement automatisé.

11. Procédé selon l'une des revendications 1 à 10, **caractérisé en ce que**, à la place d'une simple électrode pour la détection, on utilise une électrode structurée, par exemple une électrode interdigitale.

12. Procédé selon l'une des revendications 1 à 11, **caractérisé en ce que**, à la place d'une simple réaction redox électrochimique, on utilise une réaction cyclique catalysée enzymatiquement.
